# EUROPEAN PATENT APPLICATION

(11) **EP 4 512 305 A1**
(43) Date of publication of application: **26.02.2025**
(21) Application number: 23792095.4
(22) Date of filing: 12.04.2023
(51) Int. Cl.: A61B 3/024, A61B 3/00, A61B 90/00

(54) **METHOD AND APPARATUS FOR EXAMINING DEFECTS AND DEFORMITIES OF CENTRAL VISUAL FIELD**

(30) Priority: 18.04.2022 KR 20220047831
(71) Applicant: Samsung Life Public Welfare Foundation, Seoul 04348 (KR)
(72) Inventor: KANG, Se Woong, Seoul 06351 (KR); KIM, Yun Taek, Seoul 06597 (KR)
(74) Representative: Ostertag & Partner Patentanwälte mbB
(86) International application number: PCT/KR2023/004943
(87) International publication number: WO 2023/204517

(57) **Abstract**

The present invention relates to an apparatus and a method for examining defects and deformities of the central visual field. The present invention may comprise: a display on which an examination image divided into a plurality of examination regions by reference lines is shown and in which examination patterns are shown in the examination regions; an input device to which pattern recognition values of an examinee's recognition of the examination patterns are input; and a control device that derives an abnormal region of the examinee on the basis of the pattern recognition values and preset values.

## Description

### [Technical Field]

The present invention relates to an examination apparatus and method for measuring defects and deformities of a central visual field.

### [Background Art]

The macula is the most important part of the retina, which is the inner nerve layer of the eye, is the central part of the retina with a radius of about 1.5 mm, and is densely packed with photoreceptors that can sense light.

The macula is the central part of the retina and is responsible for most of our vision, and macular degeneration is a disease that causes visual impairment due to changes in macular region, located at the central part of the retina inside the eye.

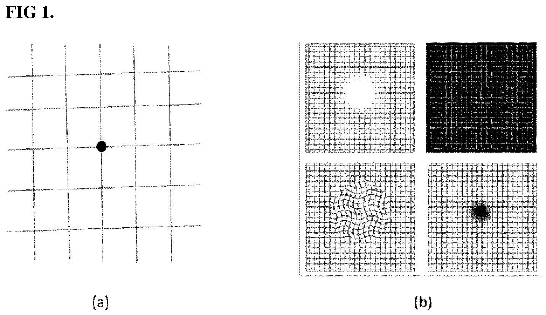

As shown in FIGS. 1A and 1B, the Amsler grid chart is generally used to examine changes in the central visual field, and symptoms such as a central dot not being visible, a grid pattern appearing warped, one side of the Amsler grid chart appearing blurry, a black spot appearing in the center of the vision, or inability to distinguish colors due to abnormalities in the macular may appear.

However, in examinations of central visual field, it is important to focus on the center for an accurate examination, but since the Amsler grid is square, the Amsler grid does not match the circular macular structure (optic nerve fiber layer, etc.), and thus it is difficult to focus on the center, which makes results of the examination unclear.

### [Disclosure]

### [Technical Problem]

The present invention is directed to providing an apparatus and method capable of examining defects and deformities of an examinee's central visual field using a pattern consisting of circles or arcs.

### [Technical Solution]

One aspect of the present invention provides an apparatus for examining defects and deformities of a central visual field, which includes a display on which an examination image divided into a plurality of examination regions by reference lines is displayed, wherein examination patterns are displayed in the examination regions, an input device to which a pattern recognition value of an examinee's recognition of the examination patterns are input, and a control device configured to derive an abnormal region of the examinee's central visual field on the basis of the pattern recognition values and preset values.

In the technology described below, the apparatus may further include a terminal from which information on the abnormal region is output.

In the technology described below, the examination regions may include quadrants formed by intersection of the reference lines, and concentric circles displayed on the quadrants to be spaced a predetermined interval from an intersecting point of the reference lines as a center.

In the technology described below, the examination patterns may include a central point displayed at the intersecting point, and an examination circle or examination arc displayed along at least one of the concentric circles.

The examination arc may be displayed along the concentric circles which are displayed on at least one of the quadrants.

In the technology described below, the examination patterns may have a lower brightness than the concentric circles.

In the technology described below, the pattern recognition values may correspond to whether the examination patterns are bent.

In the technology described below, the apparatus may further include a camera configured to photograph an eye of the examinee to obtain eye image data of the examinee, wherein the control device may compare size information of a cornea of the examinee that is included in the eye image data with data standardized for sizes of corneas according to human body information, and obtain distance information between an eye disease examination apparatus and the eye of the examinee.

Further, the control device may guide an appropriate distance for eye disease testing using the obtained distance information.

Another aspect of the present invention provides a method of examining defects and deformities of a central visual field, which includes displaying an examination image divided into a plurality of examination regions by reference lines, displaying examination patterns in the examination regions; inputting pattern recognition values of an examinee's recognition of the examination patterns, determining whether the pattern recognition values match preset values, and deriving an abnormal region of the examinee's central visual field on the basis of a result of the determination.

In the technology described below, the displaying of the examination patterns in the examination regions may include blinking a center point displayed at an intersecting point of the reference lines, displaying an examination arc on concentric circles displayed on at least one of quadrants formed by intersection of the reference lines, and displaying an examination circle on at least one of the concentric circles.

In the technology described below, in the deriving of the abnormal region of the central visual field, when the pattern recognition values do not match the preset values, it may be firstly determined that the examination arc lastly displayed in the examination regions is the abnormal region, and then the method may return to the displaying of the examination circle in the at least one of the concentric circles, or it may be secondarily determined that the examination circle lastly displayed in the examination regions is the abnormal region, and then it may be finally determined that an overlapping region between the first and second determinations is the abnormal region.

In the technology described below, in the deriving of the abnormal region of the central visual field, when the pattern recognition values match the preset values, the method may return to the displaying of the examination arc on the concentric circle, or the examinee's vision may be determined to be normal.

### [Advantageous Effects]

In an apparatus and method for examining defects and deformities of a central visual field according to the present invention, an examination pattern consisting of circles or arcs having a center on which an examinee easily focuses is displayed on a display, and the examinee's macular degeneration region can be derived for each region, thereby increasing the accuracy of examination results.

### [Description of Drawings]

FIGS. 1A and 1B sequentially show the Amsler grid, and symptoms of defects and deformities of a central visual field.
FIG. 2 is a block diagram of an apparatus for examining defects and deformities of a central visual field according to an embodiment of the present invention.
FIG. 3 is a block diagram of a control device shown in FIG. 2.
FIGS. 4A to 4D are diagrams sequentially showing a first embodiment of an examination pattern displayed on a display of FIG. 2.
FIG. 5A is a set of diagrams sequentially showing a second embodiment of the examination pattern displayed on the display of FIG. 2.
FIG. 5B is a set of diagrams showing a third embodiment of the examination pattern displayed on the display of FIG. 2.
FIG. 5C is a set of diagrams showing a fourth embodiment of the examination pattern displayed on the display of FIG. 2.
FIG. 6 is a flowchart showing a method of examining defects and deformities of a central visual field using the apparatus for examining the defects and deformities of the central visual field shown in FIG. 2 in a time series.
FIG. 7 is a flowchart showing an operation of displaying an examination pattern shown in FIG. 6 in a time series.
FIG. 8 is a flowchart showing an operation of deriving a macular degeneration region shown in FIG. 6 in a time series.

### [Best Mode of the Invention]

In order to describe the technical scope of the present invention in detail to an extent that can be easily performed by those skilled in the art, the most exemplary embodiment of the present invention will be described with reference to the accompanying drawings.

First, in this specification, when components of each drawing are given reference numerals, it should be noted that the same components are given the same reference numerals whenever possible, even when the same components are illustrated in different drawings.

Further, in description of the present invention, when it is determined that detailed descriptions of related well-known configurations or functions unnecessarily obscure the gist of the present invention, the detailed descriptions thereof will be omitted.

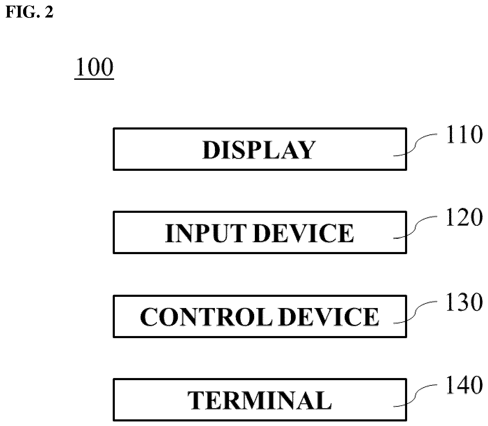

Hereinafter, an apparatus for examining defects and deformities of a central visual field according to an embodiment of the present invention will be described with reference to FIGS. 2 to 8.

First, the term "vision" according to embodiments of the present invention includes the ability of the eye to recognize the existence or shape of an object or the ability of the eye to distinguish between two points of light, and should be understood as a broad concept that includes contrast sensitivity, peripheral vision, peripheral field of vision, light detection ability, etc. That is, the term "vision examination" of the present invention is not limited to the visual acuity, which refers to a numerical value such as "1.0" or "0.8" derived from an examination to determine how large an object the eye can see, as measured at an ophthalmologist, optical shop, or physical examination, and should be understood as a concept that includes a visual field examination and an eye disease examination that can detect abnormalities in the cornea, lens, fundus, and optic nerve.

Therefore, it would be desirable to understand that an examination apparatus 100, which is referred to as a vision examination apparatus, that is, an apparatus for examining defects and deformities of a central visual field, or the like, includes any equipment that can detect diseases through an examination of vision or visual field of an examinee, such as an eye disease examination apparatus, a visual field examination apparatus, and the like.

Referring to FIG. 2, the apparatus 100 for examining defects and deformities of a central visual field may include a display 110 on which an examination image 10 divided into a plurality of examination regions 11, in which examination patterns 20 are displayed, by reference lines is displayed.

In addition, the apparatus 100 for examining defects and deformities of the central visual field may include an input device 120 into which pattern recognition values of an examinee's recognition of the examination patterns 20 are input, a control device 130 that derives an abnormal region of the examinee's central visual field on the basis of the pattern recognition values and preset values, and a terminal 140 on which the abnormal region is output.

Abnormal regions of a central visual field refer to regions formed by diseases that occur due to deformities, defects, or the like occurring in the central visual field. That is, the abnormal regions of the central visual field may refer to regions formed by various diseases including macular degeneration, epiretinal membrane, macular hole glaucoma, etc. that cause deformities, defects, or the like in the central visual field.

A known display 110 such as a light-emitting diode (LED) display may be used as the display 110, but the present invention is not limited thereto, and a known terminal 140 including the display 110, such as a smartphone, may also be used as the display 110.

The display 110 may be disposed at a location facing the examinee and may be fixed to a separate stand and disposed vertically on the ground.

The preset values may correspond to the original state of the examination patterns 20 displayed in the examination regions 11, and may be input to the control device 130 by an examiner and preset.

The pattern recognition values may correspond to whether the examination patterns are bent, and the input device 120 is a component in which the pattern recognition values are input, and may be an input unit including components such as a key pad, a touch pad, and the like.

That is, when the examinee recognizes that the examination patterns are bent or the color of the examination patterns are changed, it may be determined that the pattern recognition values do not match the preset values, and conversely, when the examinee recognizes that the examination patterns are not bent and the color of the examination patterns are not changed, it may be determined that the pattern recognition values match the preset values.

The examinee may directly input the pattern recognition values to the input device 120 or the examiner may receive the pattern recognition values through the examinee's voice and input the pattern recognition values to the input device 120.

The terminal 140 is a terminal of an examinee or examiner, and may correspond to a device including any type of handheld-based wireless communication devices such as a personal communications service (PCS) terminal, a Global System for Mobile communications (GSM) terminal, a Personal Digital Cellular (PDC) terminal, a Personal Handy-phone System (PHS) terminal, a personal digital assistant (PDA), an International Mobile Telecommunications (IMT)-2000 terminal, a Code-division multiple access (CDMA)-2000 terminal, a W-CDMA terminal, a wireless broadband internet (WiBro) terminal, a smartphone, a smart pad, a tablet PC (personal computer), etc., and a computing device such as a desktop PC or a notebook computer.

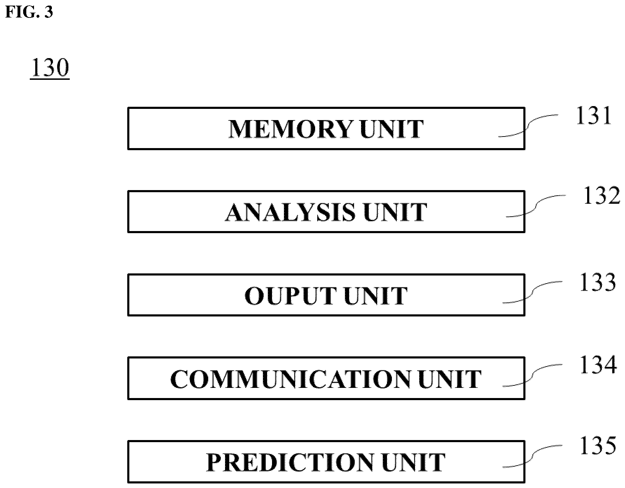

FIG. 3 is a block diagram of the control device shown in FIG. 2.

Referring to FIG. 3, the control device 130 may include a memory unit 131 in which the examination image 10, the examination patterns 20, the pattern recognition values, and the preset values are stored, an analysis unit 132 that compares the pattern recognition values with the preset values to derive an abnormal region of the central visual field, an output unit 133 that displays the examination patterns 20 on the display 110 on the basis of the preset values, and a communication unit 134 that transmits information on a macular degeneration region to the terminal 140.

The communication unit 134 may allow the components of the apparatus 100 for examining defects and deformities of the central visual field, which include the display 110, the input device 120, and the terminal 140, to be interconnected with via a network, and the network may be configured regardless of its networking mode, such as wired or wireless.

For example, the network may include various communication networks such as a local area network (LAN), a metropolitan area network (MAN), a wide area network (WAN), and the like. Preferably, the communication network referred to in the technology described below may include the Internet, which is interconnected between countries around the world.

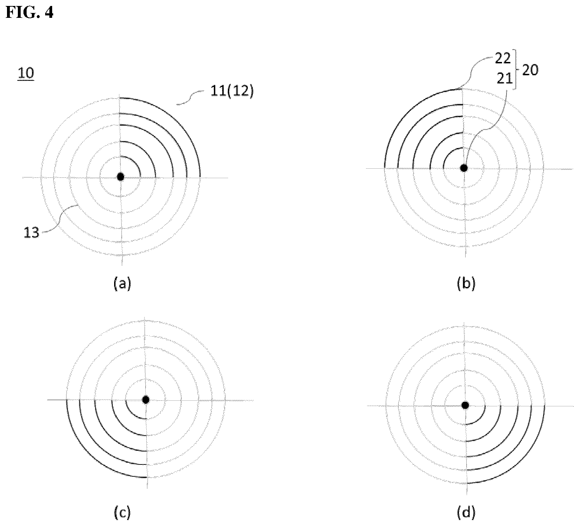

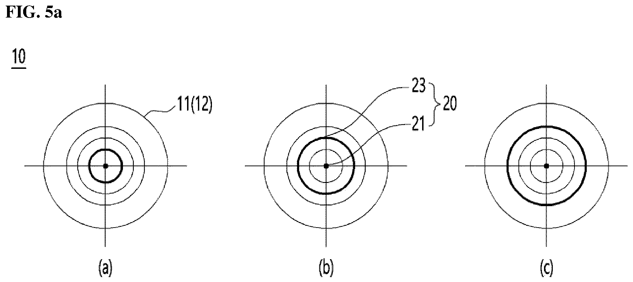

FIGS. 4A to 4D are diagrams sequentially showing a first embodiment of the examination pattern displayed on the display of FIG. 2, and FIGS. 5A(a) to 5A(c) are diagrams sequentially showing a second embodiment of the examination pattern displayed on the display of FIG. 2.

A quadrant 12 is one of four parts formed by dividing a part along two mutually orthogonal straight lines intersecting each other, and may be displayed on the display. In the quadrant 12, a central point 21 may be disposed at an intersecting point of vertical and horizontal lines of the quadrant 12, and a plurality of concentric circles 13 spaced a predetermined interval from each other may be disposed around the central point 21. A concentric circle-type Amsler grid, in which concentric circles 13 and a central point 21 are disposed as described above, may focus more easily on the central point 21 than the existing grid-type Amsler grid, thereby improving the accuracy in determination of a defect and deformity region of the central visual field.

The plurality of concentric circles 13 may be arranged in the form in which diameters thereof gradually increase as the concentric circles 13 are away from the central point 21.

Examination arcs 22 may be displayed along the plurality of concentric circles 13 on at least one of the quadrants 12 or in any arbitrary region.

The central point 21 may be preset in the control device 130 to be controlled in a manner that is not limited thereto, for example, in a manner of blinking, increasing in size, or changing in color in a gradation manner. The central point 21 may be highlighted on the quadrant 12 in the same manner as described above, which may concentrate the examinee's focus on the central point 21, thereby improving the accuracy of results of the examination.

The examination pattern 20 may be a predetermined pattern formed using the central point 21 and the examination arcs 22 or the central point 21 and examination circles 23 that are displayed on the terminal 140 in order to determine the defect and deformity region of the central visual field.

The examination pattern 20 may be displayed on at least one of the quadrants 12 or in the concentric circles 13 in any arbitrary region, along the concentric circle 13, and the color of lines of the examination pattern 20 may be an achromatic color or a chromatic color. The color of lines of the concentric circles 13 may also be an achromatic color or a chromatic color. When the color of the lines of the examination pattern 20 is an achromatic color, the color of the lines of the concentric circles 13 may also be an achromatic color of the same series, and in the case, the brightness of the examination pattern 20 is displayed to be lower than the brightness of the concentric circles 13 so that the examination pattern 20 is displayed more prominently than the concentric circles 13, and thus the examinee may recognize the examination pattern 20 more easily. When the color of the lines of the examination pattern 20 is a chromatic color, the color of the lines of the concentric circles 13 may also be a chromatic color of the same series, and in the case, the brightness of the examination pattern 20 is displayed to be lower than the brightness of the concentric circles 13 so that the examination pattern 20 is displayed more prominently than the concentric circles 13, and thus the examinee may recognize the examination pattern 20 more easily.

Further, the thickness of the lines of the examination pattern 20 is displayed to be greater than the thickness of the lines of the concentric circles 13, and thus the examinee may recognize the examination pattern 20 more easily.

The control device 130 may perform a two-stage determination process to increase the accuracy of determination of a macular degeneration region. First, a first determination may be made based on the examination arcs 22, and then a second determination may be made based on the examination circles 23.

In addition, the control device 130 may finally determine an overlapping region between the first and second determinations as a defect and deformity region of the central visual field.

In the first embodiment of the examination patterns 20, one of the quadrants 12 may be arbitrarily selected as the examination arcs 22, and the examination arcs 22 may be displayed along the concentric circles 13 displayed on the selected quadrant.

Alternatively, as shown in FIGS. 4A to 4D, respective quadrants from a first quadrant to a fourth quadrant may be sequentially selected from among the quadrants 12, and the examination arcs 22 may be displayed along the concentric circles 13 displayed on the selected quadrant.

Each time the examination arcs 22 are displayed on each quadrant, the examinee may determine whether the examination arcs 22 are bent, and the examination arcs 22 that are determined to be bent may be determined as the defect and deformity region of the central visual field.

In the second embodiment of the examination patterns 20, one of the plurality of concentric circles 13 may be arbitrarily selected as the examination circle 23, and the examination circle 23 may be displayed on the selected circle.

Alternatively, as shown in FIGS. 5A(a) to 5A(c), the concentric circles 13 may be selected in order of increasing diameter based on the central point 21, and the examination circle 23 may be displayed on the selected concentric circles 13.

Each time the examination circle 23 is displayed on each of the concentric circles 13, the examinee may determine whether the examination circle 23 is bent, and the examination circle 23 that is determined to be bent may be determined as an abnormal region.

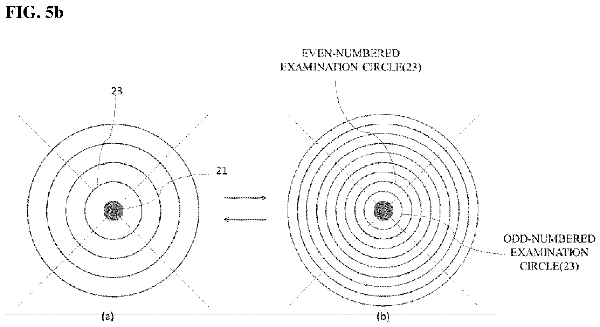

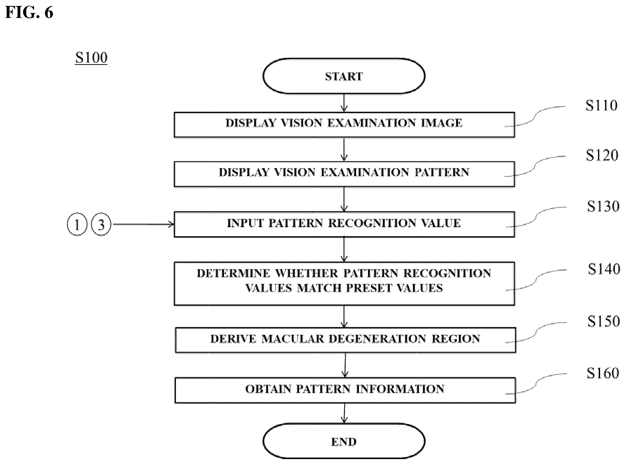

FIG. 5B shows a third embodiment of the examination patterns 20, and referring to FIG. 6, the color of lines of the examination circles 23 may be a chromatic color, and the colors of odd-numbered examination circles 23 and even-numbered examination circles 23 from the central point 21 may be displayed differently. Further, after the odd-numbered examination circles 23 are displayed, the even-numbered examination circle 23 of a different color from the odd-numbered examination circle 23 may be displayed in such a way that it is combined with the odd-numbered examination circle 23. The concentric circles 13 may be an achromatic color or a chromatic color, and the brightness of the concentric circles 13 may be displayed to be higher than the brightness of the examination circles 23 so that the examination circles 23 may be displayed to be more highlighted. Using the above method, it is possible to determine whether the examination circles 23 appear to be bent to determine whether there is metamorphopsia, and it is possible to determine whether the color of a specific region of the examination circles 23 appears to be changed to determine whether there is dichromatic vision. Further, since two different colors are used for the examination circles 23, it is also possible to determine whether there is dichromatic vision for each color.

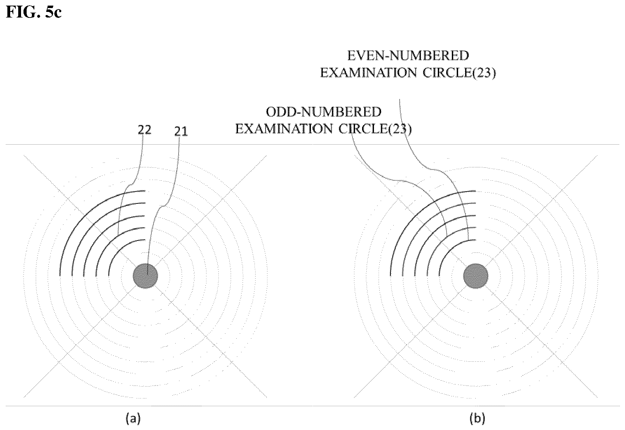

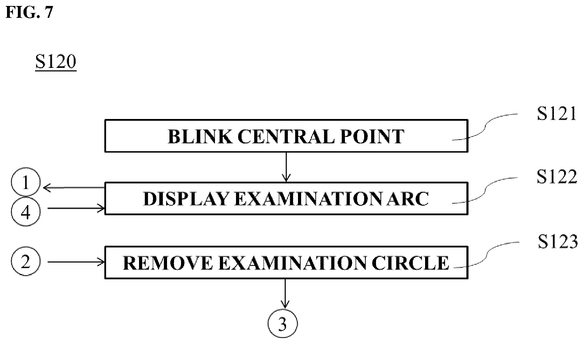

FIG. 5C shows a fourth embodiment of the examination patterns 20, and referring to FIG. 7, the color of lines of the examination arcs 22 may be a chromatic color, and the colors of odd-numbered examination circles 23 and even-numbered examination circles 23 from the central point 21 may be displayed differently. After the central point 21 and the plurality of concentric circles 13 are displayed on the quadrants 12, chromatic examination arcs 22 may be displayed on the concentric circles 13 in an arbitrary region on the quadrants 12. Using the above method, it is possible to determine whether the examination arcs 22 of the displayed region appear to be bent to determine whether there is metamorphopsia, and it is possible to determine whether the color of a specific region of the examination arcs 22 appears to be changed to determine whether there is dichromatic vision. Further, since two different colors are used for the examination arc 22, it is also possible to determine whether there is dichromatic vision for each color.

In order to identify defect and deformity patterns of the examinee's central visual field, the control device 130 may further include a prediction unit 135, and the prediction unit 135 may extract coordinate values of the defect and deformity region of the central visual field based on a certain period of time, and repeatedly train the same to obtain pattern information on the examinee's macular degeneration.

More specifically, the prediction unit 135 sets examination sections for each period of time, extracts the coordinate values of the defect and deformity region of the central visual field, and divides the extracted coordinate values into examination sections.

For example, in order to extract the defect and deformity pattern of the examinee's central visual field, the examination sections are set by dividing one year into certain periods of time, and the coordinate values of the defect and deformity region of the central visual field are repeatedly extracted for each examination section.

That is, the coordinate values of the defect and deformity region of the central visual field extracted during a certain period of time may be identified and repeatedly trained, and the defect and deformity pattern of the examinee's central visual field may be inferred based on the training so that the pattern information may be obtained.

In addition, by comparing the pattern information with the coordinate values of the defect and deformity region of the central visual field collected in real time, it is possible to predict a time and region where the defects and deformities of the central visual field may occur in the future.

Accordingly, by performing vision correction in advance for the defect and deformity region of the central visual field, the worsening of the condition may be prevented, or the examinee's examination date may be scheduled in advance.

Further, the apparatus 100 for examining defects and deformities of the central visual field according to the embodiment of the present invention, that is, an eye disease examination apparatus or a vision examination apparatus, may be implemented as the terminal 140 itself, and when the apparatus 100 for examining defects and deformities of the central visual field is implemented as the terminal 140, the display 110, the input device 120, and the control device 130 of FIG. 1 may be included as components constituting the terminal 140.

Meanwhile, in order to perform an eye disease examination according to the embodiment of the present invention, the examinee's eye and the vision examination apparatus should be separated from each other by a certain distance. In this case, in the case of a specific place where medical professionals such as an ophthalmology department provide medical services, the examiner may be guided on the distance between the eye and the vision examination apparatus and the placement thereof, but when the examiner uses the vision examination apparatus through self-diagnosis, guidance on a certain distance is required.

According to the embodiment of the present invention, the apparatus for examining the defects and deformities of the central visual field may further include a camera that obtains image data for the examinee's face to measure a distance between the examinee's eye and the terminal.

Specifically, the camera may obtain image data for the eyes among the examinee's face.

The control device according to the embodiment of the present invention may compare the size of a cornea included in input examinee information with the size of a cornea included in image data for the photographed eye on the basis of data standardized for sizes of corneas according to human body information according to the human body information (including age, gender, etc.) to calculate a current distance between the examination apparatus and the examiner's eye.

In this case, the size of the cornea refers to the white to white distance (WTW or cornea (1) distance) of the eye, and may be a horizontal corneal diameter measured between limbal borders.

Thereafter, the control device may calculate an appropriate distance by utilizing the collected user's vision, cognitive ability, age, etc., and when the current distance between the terminal and the examiner's eye is different from the appropriate distance, a screen may be output through the display to guide the appropriate distance. The vision examination apparatus according to the embodiment of the present invention may be a terminal of an examinee or examiner, as described above, and the user corresponding to the examinee or examiner may perform self-diagnosis of eye diseases using the vision examination apparatus by himself/herself at the appropriate distance.

In this case, the self-diagnosis may be implemented as an application installed inside the vision examination apparatus.

Hereinafter, a method of examining defects and deformities of a central visual field using the above-described apparatus for examining defects and deformities of the central visual field will be described.

FIG. 6 is a flowchart showing in a time series a method of examining defects and deformities of a central visual field using the apparatus for examining the defects and deformities of the central visual field shown in FIG. 2, and FIG. 7 is a detailed flowchart of an operation of displaying the examination patterns shown in FIG. 6.

Referring to FIGS. 6 and 7, in the method of examining defects and deformities of the central visual field (S100), first, the control device 130 displays the examination image 10 on the display 110 (S110), and displays the examination patterns 20 in the examination regions 11 (S120).

More specifically, the control device 130 blinks the central point 21 so that the examinee's gaze is focused on the central point 21 (S121), and displays the examination arcs 22 on the concentric circles 13 displayed on at least one of the quadrants 12 (S122).

Thereafter, the input device 120 may receive pattern recognition values for the examination patterns 20 (S130).

Thereafter, the control device 130 determines whether the pattern recognition values match preset values (S140), and derives a defect and deformity region of the examinee's central visual field on the basis of a result of the determination (S150).

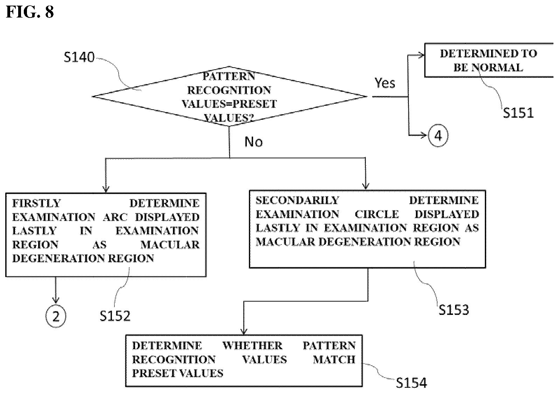

FIG. 8 is a detailed flowchart of an operation of deriving the defect and deformity region of the central visual field shown in FIG. 6.

Referring to FIG. 8, more specifically, when the pattern recognition values match the preset values, the control device 130 may display the examination arcs 22 on the concentric circles 13 displayed on another one of the quadrants (S122).

Here, when the pattern recognition values match the preset values and the quadrant where the examination arcs 22 are displayed is a fourth quadrant among the quadrants 12, the entire examination regions is considered to have been examined, and the examinee's vision is determined to be normal (S151).

Alternatively, when the pattern recognition values do not match the preset values, the control device 130 firstly determines the examination arcs 22 displayed lastly in the examination regions 11 as the defect and deformity region of the central visual field (S152).

Thereafter, the control device 130 may display the examination circles 23 on at least one of the concentric circles 13 (S123), and the input device 120 may re-receive the pattern recognition values (S130).

Thereafter, the control device 130 re-determines whether the pattern recognition values match the preset values (S140), and derives the defect and deformity region of the examinee's central visual field on the basis of a result of the determination (S150).

Here, when the pattern recognition values do not match the preset values, it is secondarily determined that the examination circles 23 displayed lastly in the examination regions 11 is a macular degeneration region (S153), and then it is finally determined that an overlapping region between the first and second determinations is the defect and deformity region of the central visual field (S154).

Alternatively, when the pattern recognition values match the preset values, the examination circles 23 may be displayed on another one of the concentric circles 13 (S123), and the process of inputting the pattern recognition values may be repeated (S130).

Thereafter, the control device 130 obtains pattern information, compares the pattern information with coordinate values of the defect and deformity region of the central visual field collected in real time, and predicts a time and region where the defects and deformities of the central visual field may occur in the future (S160).

As described above, in the apparatus and method for examining defects and deformities of the central visual field according to embodiments of the present invention, examination patterns consisting of circles or arcs having a center on which an examinee can easily focus can be displayed on a display, a defect and deformity region of an examinee's central visual field can be derived for each region, and thus the accuracy of examination results can be increased.

The best embodiment has been disclosed in this specification with reference to the accompanying drawings. Although specific terms are used herein, these terms are used only for the purpose of describing the present invention and are not intended to limit the meaning or the scope of the present invention as defined in the appended claims. Therefore, it should be understood by those skilled in the art that various alterations and equivalent other embodiments may be made. Therefore, the scope of the present invention is defined by the appended claims.

## Claims

1. An apparatus for examining defects and deformities of a central visual field, comprising:
a display on which an examination image divided into a plurality of examination regions by reference lines is displayed, wherein examination patterns are displayed in the examination regions;
an input device to which a pattern recognition value of an examinee's recognition of the examination patterns are input; and
a control device configured to derive an abnormal region of the examinee's central visual field on the basis of the pattern recognition values and preset values.

2. The apparatus of claim 1, further comprising a terminal from which information on the abnormal region is output.

3. The apparatus of claim 1, wherein the examination regions include:
quadrants formed by intersection of the reference lines; and
concentric circles displayed on the quadrants to be spaced a predetermined interval from an intersecting point of the reference lines as a center.

4. The apparatus of claim 3, wherein the examination patterns include:
a central point displayed at the intersecting point; and
an examination circle or examination arc displayed along at least one of the concentric circles.

5. The apparatus of claim 4, wherein the examination arc is displayed along the concentric circles which are displayed on at least one of the quadrants.

6. The apparatus of claim 3, wherein the examination circle or examination arc has a chromatic color.

7. The apparatus of claim 6, wherein the examination patterns are displayed with different brightness or color from the concentric circles.

8. The apparatus of claim 7, wherein a color of an odd-numbered examination circle or arc from a central point is different from that of an even-numbered examination circle or arc from the central point.

9. The apparatus of claim 3, wherein the pattern recognition value indicates whether the examination pattern is bent and whether a color of the examination pattern is changed.

10. The apparatus of claim 1, further comprising a camera configured to photograph an eye of the examinee to obtain eye image data of the examinee,
wherein the control device compares size information of a cornea of the examinee that is included in the eye image data with data standardized for sizes of corneas according to human body information, and obtains distance information between an eye disease examination apparatus and the eye of the examinee.

11. The apparatus of claim 10, wherein the control device guides an appropriate distance for eye disease testing using the obtained distance information.

12. A method of examining defects and deformities of a central visual field, comprising:
displaying an examination image divided into a plurality of examination regions by reference lines;
displaying examination patterns in the examination regions;
inputting pattern recognition values of an examinee's recognition of the examination patterns;
determining whether the pattern recognition values match preset values; and
deriving an abnormal region of the examinee's central visual field on the basis of a result of the determination.

13. The method of claim 12, wherein the displaying of the examination patterns in the examination regions includes:
highlighting a center point displayed at an intersecting point of the reference lines;
displaying an examination arc on concentric circles displayed on at least one of quadrants formed by intersection of the reference lines; and
displaying an examination circle on at least one of the concentric circles.

14. The method of claim 13, wherein, in the deriving of the abnormal region, when the pattern recognition values do not match the preset values, it is firstly determined that the examination arc lastly displayed in the examination regions is the abnormal region, and then the method returns to the displaying of the examination circle in the at least one of the concentric circles, or it is secondarily determined that the examination circle lastly displayed in the examination regions is the abnormal region, and then it is finally determined that an overlapping region between the first and second determinations is the abnormal region.

15. The method of claim 13, wherein, in the deriving of the abnormal region, when the pattern recognition values match the preset values, the method returns to the displaying of the examination arc on the concentric circle, or the examinee's vision is determined to be normal.

16. The method of claim 12, further comprising:
photographing an eye of the examinee using a camera and obtaining eye image data of the examinee; and
comparing size information of a cornea of the examinee that is included in the eye image data with data standardized for sizes of corneas according to human body information, and obtaining distance information between an eye disease examination apparatus and the eye of the examinee.

17. The method of claim 16, further comprising guiding an appropriate distance for eye disease testing using the obtained distance information.
